Europäisches Patentamt

(19) European Patent Office

Office européen des brevets

(11) Publication number: **0 142 790**
**A2**

# (12) EUROPEAN PATENT APPLICATION

(21) Application number: **84113524.7**

(22) Date of filing: **09.11.84**

(51) Int. Cl.⁴: **C 12 P 9/00**
**A 61 K 33/26**
**//C12R1/645, C12R1/85,**
**C12R1/865, C12P9:00**

(30) Priority: **11.11.83 JP 210964/83**

(43) Date of publication of application:
**29.05.85 Bulletin 85/22**

(84) Designated Contracting States:
**AT BE CH DE FR GB IT LI LU NL SE**

(71) Applicant: **Tanaka, Kentaro**
**1-26, Misaki 1-chome Kofu-shi**
**Yamanashi-ken(JP)**

(72) Inventor: **Tanaka, Kentaro**
**1-26, Misaki 1-chome Kofu-shi**
**Yamanashi-ken(JP)**

(74) Representative: **Strehl, Schübel-Hopf, Schulz**
**Widenmayerstrasse 17 Postfach 22 03 45**
**D-8000 München 22(DE)**

(54) An antianaemic composition and a process for producing the same.

(57) An improved process for producing an organoiron (II) compound-containing antianaemic composition which comprises subjecting a yeast to anaerobic culturing in a saccharide-containing nutrient medium therefor in the presence of an iron compound. By the process, an antianaemic composition which is very stable and excellent in absorbability into a living body and incorporation of iron into hemoglobin can be produced in high yield in a shortened period of time.

EP 0 142 790 A2

0142790

This invention relates to an antianaemic composition and a process for producing the same. More particularly, it is concerned with an organoiron (II) compound-containing antianaemic composition which is chemically stable and can be well absorbed into a living body, and the production of the same by a process comprising subjecting a yeast to culturing in a saccharide-containing nutrient medium in the presence of an iron compound.

Since iron is a major constituent of hemoglobin, various kinds of iron compounds are used as an antianaemic. Heretofore, a considerable number of researches have been made on the absorption of iron into a living body through the stomach and intestine organs. As a result, it is now known that $Fe^{++}$ can be well absorbed in the human organs but $Fe^{+++}$ is not. According to the research of Brise and Hallberg disclosed in Acta Med. Scandinar, vol. 171 suppl. 376, pp23-38 (1962), the absorbability of each of iron salts into the human body, assuming that the absorbability of ferrous sulfate is 100, is as follows:

| | |
|---|---|
| ferrous succinate | 120 - 157 |
| ferrous lactate | 90 - 128 |
| ferrous fumarate | 100 - 105 |
| glycinated ferrous sulfate | 87 - 110 |

- 2 -

| | |
|---|---|
| ferrous glutamate | 50 - 118 |
| ferrous gluconate | 68 - 110 |
| ferrous citrate | 60 - 90 |
| ferrous tartrate | 45 - 87 |
| ferrous pyrophosphate | 46 - 80 |
| cholinated ferric isocitrate | 25 - 50 |
| ferric sulfate | 27 - 50 |
| ferric citrate | 23 - 47 |
| ferric ethylenediaminetetraacetate | 16 - 33. |

Accordingly, divalent iron compounds such as ferrous sulfate and ferrous succinate are most frequently used as a chalybeate. When using such divalent iron compounds as a chalybeate, however, special pharmaceutical techniques such as coating should be applied because they are readily oxidized to trivalent iron compounds. It is known that certain iron complexes with an organic acid, chelating agent, sugar, amino acid or the like are better absorbed into a living body than inorganic iron compounds. Nowadays, an organic acid salt of a divalent iron such as ferrous fumarate, ferrous succinate or ferrous orotate, or a divalent iron chelate compound has been widely used for the treatment of a patient suffering from anaemia. Such ferrous compounds, however, still have a disadvantage that they tend to easily be oxidized to the corresponding

- 3 -

ferric compounds during the storage or in the living body even after administration thereof to a patient.

Also, wine has long been believed to be effective for the treatment of a patient suffering from anaemia. but any elucidation has not yet been made as to whether the iron values contained in wine are really effective as a chalybeate, or as to in what state the iron values in wine exert an antianaemic effect even if they are admitted to be effective. Further, there is not any accepted conviction as to where the iron values contained in wine originate. Furthermore, the iron content in the wine put on the market widely varies. Therefore, drinking wine does not always ensure absorption of iron into the living body.

The present inventor previously investigated the action of iron in various nutrient mediums inclusive of grape juice during the fermentation stage thereof and the in vivo action of iron after administration of a fermentation product to a living body. As a result of the investigation, as disclosed in European Patent Application Laid-Open Specification No. 0 078 859, it was confirmed that an amount of iron to which the iron content present in the wine put on the market can be attributed is not contained in the ordinary raw material for wine and that most of the iron values present in the wine put on the market is due to contamination during the fermentation stage.

It was also found that the soluble organoiron(II) compound contained in the composition which is obtained by culturing a yeast in a saccharide-containing nutrient medium in the presence of an iron compound is much more effective in capability of being absorbed in the living body of a human being and animals and in incorporation into hemoglobin than the conventional chalybeats and antianaemics. Based on the confirmation and finding, a process for preparing an antianaemic composition was proposed, as disclosed in the above-mentioned European patent application laid-open specification. However, that process cannot be stated as being a commercially advantageous manufacturing process, because the production yield is relatively low. Accordingly, the present inventor has further made extensive and intensive researches, and as a result has unexpectedly found that a remarkable production time saving and production yield improvement can be attained by conducting that process under an anaerobic condition. Based on this novel finding, the present invention has been completed.

Accordingly, it is an object of the present invention to provide an improved process, permitting not only the production time to be reduced but also the production yield to be increased, for producing an organoiron(II) compound-containing antianaemic composition which can be efficiently absorbed into a living body and chemically stable.

It is another object of the present invention to provide an organoiron(II) compound-containing antianaemic composition produced by a process as mentioned above.

The foregoing and other objects, features and advantages of the present invention will be apparent to those skilled in the art from the following detailed description taken in connection with the accompanying drawings in which:

Fig. 1 is a graph showing the relationship between the pH of the culture medium and the fermentation period, which will be explained later with respect to Example 1;

Fig. 2 is a graph showing the relationship between the optical absorbance of the culture medium and the fermentation period, which will be explained later with respect to Example 1;

Fig. 3 is a graph showing the relationship between the iron concentration of the culture medium and the fermentation period, which will be explained later with respect to Example 1;

Fig. 4 is a graph showing the iron concentration of each of a non-adsorptive organoiron(II) compound-containing fraction and an adsorptive organoiron(II) compound-containing fraction, which will be explained later with respect to Example 1; and

- 6 -

Fig. 5 is a graph showing the relationships between the iron contents in the living body at its plasma, bone-marrow, erythrocyte and small intestine and the lapse of time after administration of the present antianaemic composition.

In one aspect of the present invention, there is provided a process for producing an organoiron(II) compound-containing antianaemic composition which comprises subjecting a yeast to anaerobic culturing in a saccharide-containing nutrient medium therefor in the presence of an iron compound to form a cultured broth comprising an organoiron(II) compound, alcohol and water.

In practicing the present invention, any saccharide-containing nutrient medium may be employed insofar as the saccharide-containing nutrient medium is non-toxic, that is, acceptable to a living body.

Examples of the saccharide-containing nutrient medium to be employed in the present invention include a fruit juice, a semi-synthetic culture medium and a synthetic culture medium. Specific examples of the fruit juice include grape juice, apple juice and the like. Further, as the nutrient medium, there also may be employed molasses, strained lees of a sugar cane and other raw materials for sugar, strained lees of corn and the like. From the standpoint of industrial application, a semi-synthetic culture medium or a synthetic culture medium may be preferably 0142790 employed. The term "synthetic culture medium" used in the present invention means a culture medium prepared only from pure known substances which are chemically defined. As suitable examples of the synthetic culture medium, there may be mentioned the Wickerham's culture medium as given in Table 1-(1) below and the like. The term "semi-synthetic culture medium" used in the present invention means a culture medium which contains as main components carbon source, nitrogen source, and salts which are chemically pure and as the other components substances which are not necessarily chemically pure but contain minor components necessary for culturing, such as vitamins, amino acids and metals. As such substances, there may be employed, for example, yeast extract, malt extract and the like.

Preferred examples of synthetic culture medium and semi-synthetic culture medium are given in Tables 1 and 2, respectively.

Table 1 (synthetic)

(1)

| | |
|---|---|
| D-glucose | 100 g |
| Potassium sodium tartrate | 5.4 g |
| Malic acid | 2.0 g |
| $CaCl_2 \cdot 2H_2O$ | 551 mg |
| $FeCl_3 \cdot 6H_2O$ | 242 mg |

- 8 -

| | |
|---|---|
| $(NH_4)_2SO_4$ | 3.5 g |
| $KH_2PO_4$ | 1.0 g |
| $MgSO_4 \cdot 7H_2O$ | 0.5 g |
| NaCl | 0.1 g |
| $CuSO_4 \cdot 5H_2O$ | 40 µg |
| $H_3BO_3$ | 500 µg |
| KI | 100 µg |
| $MnSO_4 \cdot H_2O$ | 400 µg |
| $NaMoO_4 \cdot 2H_2O$ | 200 µg |
| $ZnSO_4 \cdot 7H_2O$ | 400 µg |
| Biotin | 10 µg |
| Calcium pantothenate | 400 µg |
| Inositol | 2000 µg |
| Thiamin·HCl | 400 µg |
| Distilled water | 1000 ml |

(Initial pH: 6.8)

(2)

| | |
|---|---|
| D-glucose | 150 g |
| $(NH_4)_2SO_4$ | 3.5 g |
| $KH_2PO_4$ | 1.0 g |
| $MgSO_4 \cdot 7H_2O$ | 0.50 g |
| NaCl | 0.10 g |
| $CaCl_2 \cdot 2H_2O$ | 0.1 g |
| $H_3BO_3$ | 500 µg |
| $CuSO_4 \cdot 5H_2O$ | 40 µg |
| KI | 100 µg |

- 9 -

| | |
|---|---|
| FeCl$_3\cdot$6H$_2$O | 242 mg |
| MnSO$_4\cdot$H$_2$O | 400 µg |
| Na$_2$MoO$_4\cdot$2H$_2$O | 200 µg |
| Biotin | 2 µg |
| ZnSO$_4\cdot$7H$_2$O | 400 µg |
| Calcium pantothenate | 400 µg |
| Inositol | 2000 µg |
| Thiamin$\cdot$HCl | 400 µg |
| Distilled water | 1000 ml |

(Initial pH:  7.0)

Table 2 (semi-synthetic)

| | |
|---|---|
| D-glucose | 100.0 g |
| (NH$_4$)$_2$SO$_4$ | 5.0 g |
| KH$_2$PO$_4$ | 1.0 g |
| MgSO$_4\cdot$7H$_2$O | 0.50 g |
| NaCl | 0.10 g |
| CaCl$_2\cdot$2H$_2$O | 0.10 g |
| Yeast extract | 1.0 g |
| Distilled water | 1000 ml |

(Initial pH:  6.0)

The kinds of the synthetic and semi-synthetic culture media, of course, are not limited to the above-mentioned examples, and any formulations employable in an ordinary fermentation using a saccharide-containing nutrient medium may be employed.

In practicing the present invention, a yeast is subjected to anaerobic culturing in a saccharide-containing nutrient medium in the presence of an iron compound. The term "anaerobic culturing" used in the present invention means culturing in which a nutrient medium is not directly contacted with air (especially with oxygen in air). By the anaerobic culturing of a yeast, gases such as carbon dioxide are formed as by-products and, therefore, if a culture vessel is sealed hermetically, the pressure in the culture vessel is increased. So, in general, there is usually employed a gas-transmitting stopper, such as cotton stopper, in the culture vessel so that the gases produced during fermentation may escape. However, a stopper like a cotton stopper causes air in the outside of a culture vessel to be transmitted into the culture vessel and, hence, an anaerobic condition cannot be obtained. In the present invention, the anaerobic condition may be obtained by sealing the culture vessel with a fermentation stopper. The term "fermentation stopper" used in the present invention means a stopper which is capable of discharging the gases produced by the anaerobic culturing but preventing transmission of gases (mainly, air) in the outside of the culture vessel into the culture vessel, that is, capable of preventing contact of the nutrient medium with the outside gases. The fermentation stopper has in its inner portion a layer containing sulfuric acid. Through the layer containing sulfuric acid, the gases produced by the anaerobic culturing are discharged. Even if the pressure in the culture vessel is reduced to a level somewhat lower than atmospheric pressure, the air in the outside of the culture vessel is not transmitted into the culture vessel by the function of the fermentation stopper. Accordingly, by the use of the above-mentioned fermentation stopper, the fermentation can be conducted while preventing not only the increase of the pressure in the culture vessel but also the direct contact of the culture medium with air, thereby enabling anaerobic culturing be effectively attained. In effecting the anaerobic culturing using the fermentation stopper for sealing the culture vessel, it is preferred that the culture vessel be allowed to stand still because the culturing operation is simple.

As mentioned above, the anaerobic culturing may be effected using a fermentation stopper. However, the method for attaining anaerobic culturing, of course, is not limited to the above-mentioned method in which a culture vessel is sealed with a fermentation stopper, and any other methods may be employed as far as they can provide an anaerobic condition.

In effecting the process of the present invention, an organic acid salt, or both an organic acid salt and calcium chloride (ordinarily in the form of $CaCl_2 \cdot 2H_2O$) may advantagously be added to a saccharide-containing nutrient medium.

As is apparent from Examples which will be given later, when anaerobic culturing of a yeast is conducted in a saccharide-containing nutrient medium, preferably synthetic culture medium or semi-synthetic culture medium, containing both an organic acid salt and calcium chloride, the yeast is proliferated to a great extent and consequently, a soluble organoiron(II) compound which is excellent in absorbability for a living body can be produced in high yield. In addition, the rate of production of the organoiron(II) compound can also be increased to a great extent and, therefore, the production time can be shortened. Further, in the nutrient medium containing an organic acid salt or both an organic acid salt and calcium chloride, the decrease of a hydrogen ion concentration of the nutrient medium can be well prevented due to a buffer action (the hydrogen ion concentration in terms of pH of the nutrient medium is not lowered to less than about 4.0). When a fruit juice is employed as the saccharide-containing nutrient medium, the fruit juice generally contains an organic acid salt and calcium chloride but the concentrations of the organic acid salt and calcium chloride are varied.  Therefore, in order to attain the above-mentioned effects of the present invention, the organic acid salt and/or calcium chloride may be added to the fruit juice so that the concentrations of the organic acid salt and calcium chloride are within the preferred range as mentioned later.

As the organic acid salt which may be employed in the present invention, any organic acid salt which is non-toxic, namely, acceptable to a living body may be employed.  Examples of the organic acid salt include malic acid salts, citric acid salts, tartaric acid salts, succinic acid salts and the like.  The above-mentioned organic acid salt may be employed either alone or in combination.  As a metal component of the above-mentioned organic acid salt,there may be mentioned, for example, sodium, potassium and the like.

In the present invention, the organic acid salt concentration of the saccharide-containing nutrient medium may be generally in the range of from about 0.1 to about 1.0 % by weight.  When the organic acid salt concentration be less than about 0.1 % by weight, the above-mentioned effects can not be sufficiently obtained.  Even if the organic acid salt concentration is more than about 1.0 % by weight, the proportional increase in effect cannot be obtained.  From the standpoints of provision of better culture conditions and economy, it is not preferable to employ an organic acid salt at a concentration more than about 1.0 %.

The calcium chloride concentration of the saccharide-containing nutrient medium may be generally in the range of from about 0.001 to about 1.0 % by weight in terms of $CaCl_2 \cdot 2H_2O$, preferably about 0.005 to about 0.1 % by weight in terms of $CaCl_2 \cdot 2H_2O$. When the calcium chloride concentration is less than about 0.001 % by weight in terms of $CaCl_2 \cdot 2H_2O$, the above-mentioned effects can not be sufficiently obtained. Even if the calcium chloride concentration is more than about 1.0 % by weight in terms of $CaCl_2 \cdot 2H_2O$, the proportional increase in effect cannot be obtained.

By the anaerobic culturing of a yeast in the saccharide-containing nutrient medium in the presence of an iron compound, a cultured broth comprising an organoiron(II) compound, alcohol and water is formed.

The cultured broth thus formed may be subjected to removal of alcohol to such an extent that the alcohol concentration of the cultured broth is decreased to less than about 1 % by volume so that the cultured broth can be safely administered to a patient suffering from anaemia for a prolonged period of time. The removal of alcohol from the cultured

- 15 -

broth may be efficiently effected by any of the commonly-known techniques, for example, using a rotary evaporator at a temperature not exceeding about 30 °C under a pressure of about 15 to 20 mmHg.

The alcohol concentration of the cultured broth may be traced by determining the specific gravity of each aliquot taken out from the cultured broth. Illustratively stated, an aliquot is taken out from the cultured broth, and the specific gravity of the aliquot at a predetermined temperature is measured. Then, the alcohol concentration corresponding to the specific gravity is read on the graph obtained by plotting the specific gravity of each solution having a known alcohol concentration against the alcohol concentration.

The method for determining the alcohol concentration is not limited to the above-explained method, but in fact any other appropriate method, including chromatography, can be employed.

The kind of a yeast to be used in the present invention is not critical, but a wide variety of genera ordinarily employed in fermentation may be employed. Preferable genus is _Saccharomyces_, _Metschnikowia_ or _Candida_. Examples of species of yeast which may preferably be employed are shown in Table 3.

Table 3

| | Genus | Species | Strain |
|---|---|---|---|
| 1. | Saccharomyces | cerevisiae | OC-2 |
| 2. | Metschnikowia | pulcherrima | K-427 |
| 3. | Saccharomyces | cheralieri | IAM-4861 |
| 4. | Saccharomyces | bayanus | PW-15 |
| 5. | Saccharomyces | italicus | PW-27 |
| 6. | Saccharomyces | fermentati | WF-107 |
| 7. | Saccharomyces | rosei | 28-t |
| 8. | Saccharomyces | heterogenicus | PW-25 |
| 9. | Candida | krusei | K-503 |

Note: The species and strains listed in Table 3 are all known.

The above-mentioned species of yeast may be employed either alone or in combination. Of the above-mentioned species, Saccharomyces cerevisiae is most preferred because it can produce a large quantity of Fraction B having an excellent absorbability through the intestine as will be explained later.

In practicing the present invention, whether the fruit juice, semi-synthetic culture medium or synthetic culture medium is used, the yeast may be employed at a yeast population of about $5 \times 10^5$ to about $3 \times 10^6$ per ml of the culture medium.

As the iron compound, any of those which can be ionized with respect to iron may be employed. As specific examples, there can be mentioned inorganic acid salts of iron such as ferric chloride, ferrous sulfate and ferric sulfate and organic acid salts of iron such as iron citrate and iron tartrate. Iron powder is also useful, but disadvantageously needs a long period of time for its dissolution into a culture medium. From the viewpoints of solubility, availability and non-toxicity, the above-mentioned iron compounds are preferred. Such an iron compound, when it is of trivalent iron, is converted to produce ferrous ions during the fermentation. The amount of the iron compound to be added is not critical, but even if the amount of iron added is increased, the amount of iron in the product is not necessarily increased in proportional relationship. Further, the addition of too laege an amount of the iron compound adversely suppresses the progress of fermentation. On the other hand, the use of too small an amount of the iron compound cannot exert the intended effect. Usually, the iron compound may be employed in the nutrient medium at a concentration of about 10 to about 70 ppm, more preferably about 30 to about 50 ppm in terms of amount of iron.

Further fermentation conditions will be given below. The kind of saccharides·to be added to the culture medium is not critical but a saccharose such as sucrose, glucose or fructose is preferably employed. The amount of a saccharide in the nutrient medium is also not critical. From a viewpoint of provision of better conditions of the desired fermentation, the saccharide may generally be incorporated into the nutrient medium in an amount of about 5 to about 25 % by weight, more preferably about 10 to about 15 % by weight based on the nutrient medium.

When the nutrient medium is a semi-synthetic culture medium or a synthetic culture medium, the anaerobic culturing may be conducted at about 18 to about 30 °C. In the anaerobic culturing of the process according to the present invention, when a nutrient medium containing an organic acid salt or both an organic acid salt and calcium chloride added thereto is used, the pH value of the culture medium rapidly decreases around two days after the initiation of the fermentation and reaches about 4.0 around three days after the initiation of the fermentation. Thereafter, the pH value does not change and is constantly about 4.0. In the case of a culture medium of fruit juice, the culturing of a yeast can be carried out according to a customary process for the production of wine, except necessity of anaerobic condition. For example, the fruit juice, e.g., grape juice or apple juice, as such, or after a saccharide such as glucose, fructose, sucrose or the like is added thereto in such an amount as will exhibit a refraction saccharide degree of about 20 to about 25, is subjected to treatment with a small amount of a germicide (e.g., about 100 ppm of potassium metabisulfite) to kill contaminating microorganisms such as wild yeast. Then, the anaerobic culturing may usually be conducted at room temperature to 30 °C.

According to the process of the present invention, there can be obtained an organoiron(II) compound-containing antianaemic composition in the form of a cultured broth by effecting alcohol fermentation through the anaerobic culturing of a yeast in a saccharide-containing nutrient medium in the presence of an iron compound. The cultured broth may be then separated into precipitate and supernatant, for example, by means of centrifugation. The obtained supernatant also is one form of an antianaemic composition produced according to the process of the present invention. Any of the obtained cultured broth and supernatant may be subjected to removal of alcohol by the above-mentioned method to such an extent that the alcohol concentration is decreased to less than about 1 % by volume. The cultured broth and the supernatant both contain a water soluble organoiron(II) compound. It is believed that the water soluble organoiron(II) compound is a fermentation product produced by the chemical change, during the culturing or fermentation, of an iron compound initially added to the culture medium. Both the cultured broth and the supernatant as such can be administered. For example,

0142790

when the culture medium is grape juice, they can be enjoyed by people as an organoiron(II)-containing wine. Alternatively, both the cultured broth and the supernatant may be concentrated at a temperature of 30 °C or lower under a reduced pressure of about 15 to 20 mmHg to obtain a ten times or higher concentrated product. The obtained concentrate may be used as a medical alcohol beverage(elixir). The concentrates may be ultimately concentrated to dryness, and the resultant as such or, if desired, together with a reducing additive such as Vitamin C, may be formulated into the adequate form for oral administration, for example, a tablet, capsule, powder, granule or fine granule form. If desired, these dosage forms may be easily prepared according to conventional technique and may comprise commonly employed excipients, binding agents, disintegrators, glidants and other pharmaceutical agents. As the excipient, binding agent and/or disintegrator, there may be, for example, mentioned microcrystalline cellulose, wheat starch, sugar, lactose, gum arabic, tragacanth gum, carboxymethylcellulose and so on. As the glidant, there may be given, e.g., magnesium stearate and talc. Tablets may be also coated according to conventional coating procedures and any commonly employable coating materials such as, for example, shellac, ethylcellulose, hydroxymethylcellulose, polyvinyl pyrrolidone, titanium dioxide and the like may be

- 21 -

favorably applied for such purposes. The dosage may vary depending upon ages, severities and body weights of patients, but the present antianaemic composition may be usually administered in a daily dose of from about 5 mg to about 100 mg in terms of amount of iron for adults, if necessary, in divided dasage forms.

From the chemical analysis, it is believed that the active ingredient of the antianaemic composition produced according to the process of the present invention is a divalent iron-containing organic complex composed only of elements Fe, C, H and O whose molecular weight is in the range of about $10^2$ to about $5 \times 10^3$. As is apparent from Experiment which will be given later, it is clearly recognized that the organoiron (II) produced according to the process of the present invention is much more effective in capability of being absorbed in the living body of a human being and animals and incorporation into hemoglobin than the conventional chalybeates. Furthermore, it is clearly recognized that the organoiron(II) in the antianaemic composition produced according to the process of the present invention is remarkably stable as compared with divalent iron compounds such as ferrous chloride, ferrous sulfate, ferrous fumarate, ferrous succinate and ferrous tartrate.

When the supernatant as mentioned before is subjected to chromatographic fractionation, for example, by means of Amberlite XAD-2(available from Rhom and Haas Co., U.S.A.), it is separated into two fractions, namely, Fraction A and Fraction B. In this connection, Fraction B has a relatively low molecular weight but a high absorbability for the living body as compared with Fbaction A. In view of the above fact, it is believed that the organoiron(II) compound produced according to the process of the present invention is composed of at least two compounds.

The iron content of blood of a patient can be increased by administering to the patient an effective amount of an organoiron(II) compound-containing antianaemic composition produced by the process of the present invention, which process is characterized by subjecting a yeast to anaerobic cultruing in a saccharide-containing nutrient medium therefor in the presence of an iron compound.

As mentioned above, the process of producing an organoiron(II) compound-containing antianaemic composition according to the present invention comprises subjecting a yeast to anaerobic culturing in a saccharide-containing nutrient medium therefor in the presence of an iron compound and, preferably additionally an organic acid salt or both an organic acid and calcium chloride. The process of the present invention is

- 23 -

extremely excellent in the yield of the organoiron(II) compound-containing composition which can be well absorbed into the living body and is chemically stable as compared with the conventional method and, therefore, very advantageous from a commercial point of view. Specifically, in the conventional method in which the anaerobic culturing is unsatisfactory, as can be seen from Comparative Example 1 which will be given later, 4.6 mg of Fraction B is obtained from 400 ml of the cultured broth. On the other hand, in the process of the present invention, as can be seen from Example 1 which will be given later, a much larger amount of Fraction B (i.e. 9.6 mg) can be obtained from 400 ml of the cultured broth.

As is apparent from the foregoing, according to the process of the present invention, a water soluble organoiron(II) compound can be obtained in high concentration. In addition, according to the process of the present invention, the period required for culturing a yeast can be shortened to 5 to 6 days, i.e. a half or less of the period required in the conventional method and, in such a shortened culture time, the maximum amount of organoiron(II) complex can be obtained.

Furthermore, according to the process of the present invention, Fraction B which is most excellent in the absorbability for the living body can be produced in a larger amount than that obtained by the 0142790
conventional method.

The present invention will be illustrated in more detail with reference to the following Examples, which should not be construed to be limiting the scope of the present invention.

Example 1

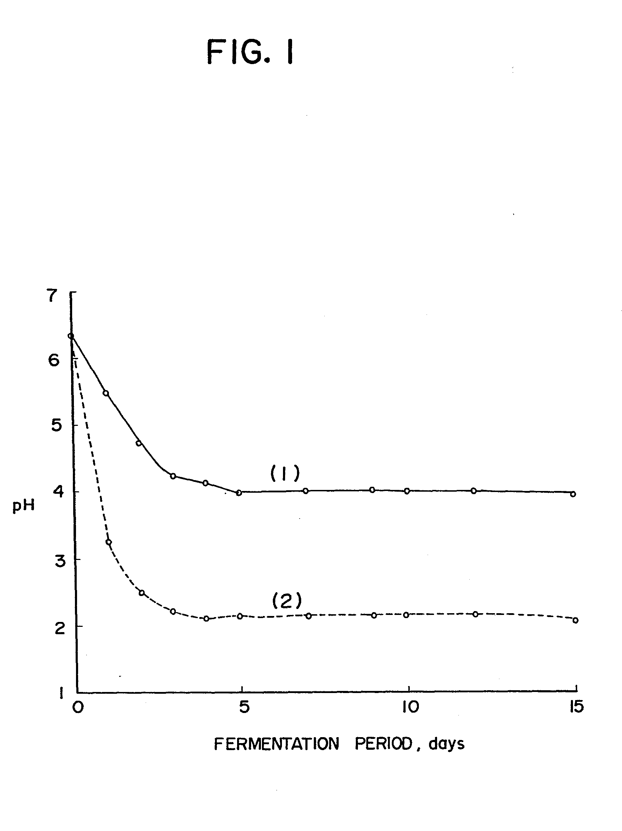

An organic acid salt-containing synthetic culture medium as indicated before in formulation (1) of synthetic culture medium given in Table 1 was put into a culture vessel. A yeast mash of Saccharomyces cerevisiae OC-2 was added to the synthetic culture medium so that the absorbance of the culture medium became 0.01(OD666) (i.e. about $4.07 \times 10^5$ cells/ml in terms of yeast population). The culture vessel was sealed with a fermentation stopper and a stationary culturing for fermentation was effected at 30 °C under atmospheric pressure. During the fermentation, the pH value, absorbance and water soluble organoiron(II) compound concentration of the culture medium were periodically measured. The results obtained are shown in the curve(1) of each of Figs. 1 to 3.

Fig. 1 shows the relationship between the pH value of the culture medium and the fermentation period. As can be seen from the curve(1) of Fig. 1, the pH value of the culture medium was about 6.8 at the initiation of the fermentation and rapidly decreased with the lapse of time. The pH value decreased to about 4.0 around three days after the initiation of the fermentation. Thereafter, there was not observed a remarkable change in the pH value.

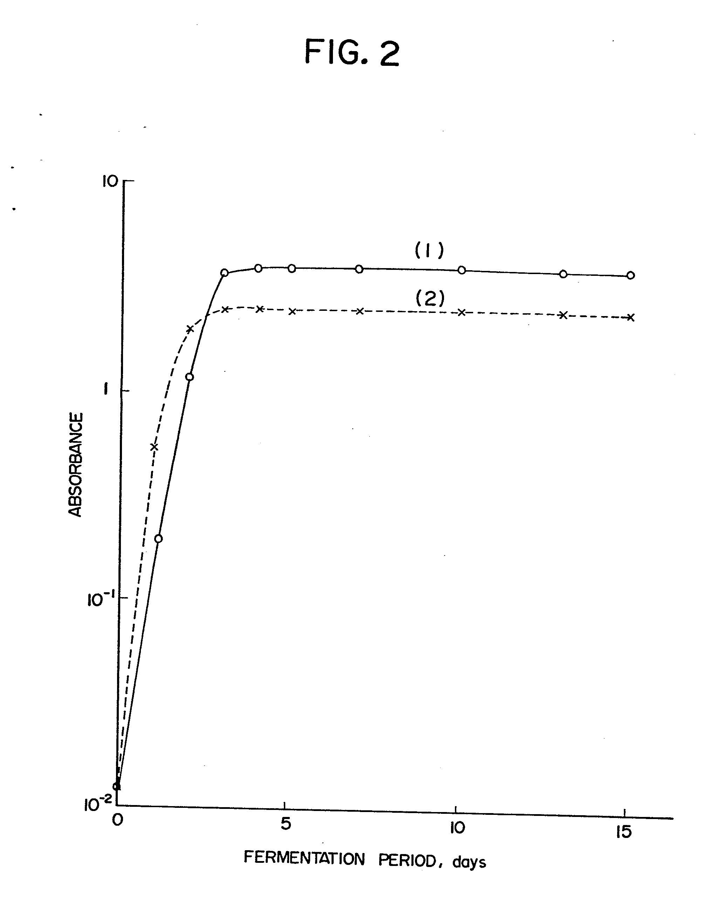

Fig. 2 shows the relationship between the absorbance of the culture medium and the fermentation period. As can be seen from the curve(1) of Fig. 2, the proliferation of Saccharomyces cerevisiae OC-2 progressed satisfactorily and major fermentation finished five days after the initiation of the fermentation.

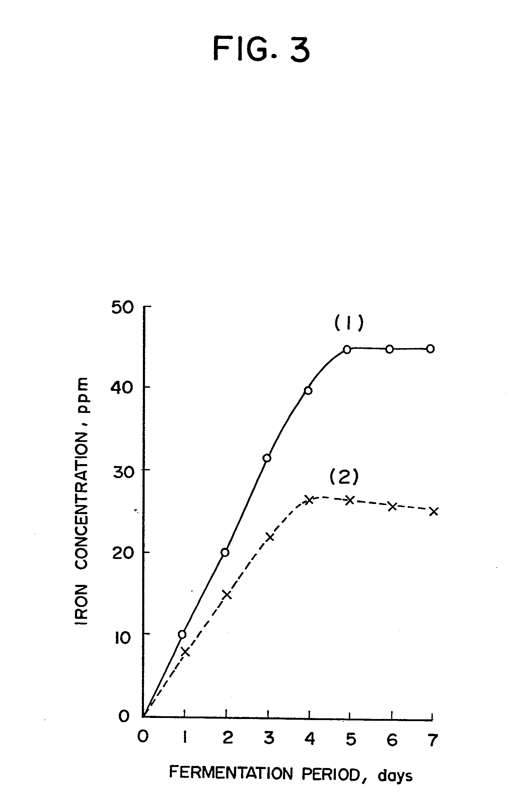

Fig. 3 shows the relationship between the water suluble organoiron(II) compound concentration of the culture medium and the fermentation period. As can be seen from the curve(1) of Fig. 3, the water soluble organoiron(II) compound concentration reached the maximum value five days after the initiation of the fermentation.

Incidentally, the curve(2) of each of Figs. 1 to 3 shows the results of fermentation in Comparative Example 2 which will be given later. The fermentation in Comparative Example 2 was effected using a cotton stopper instead of the fermentation stopper and a synthetic culture medium to which the organic acid salt (sodium potassium tartrate) and calcium chloride had not been added.

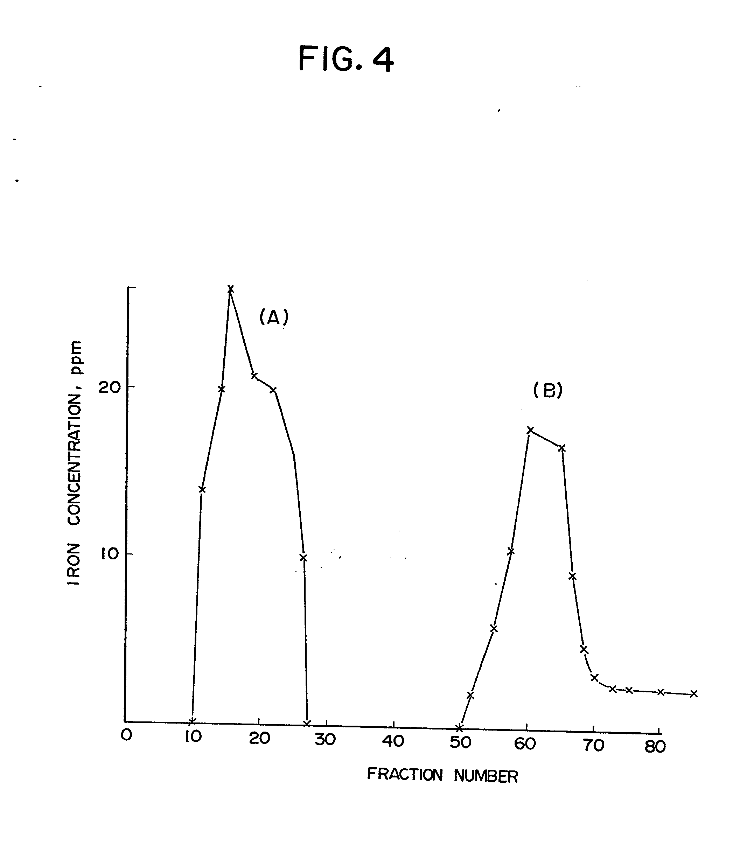

A fermentation was effected in substantially the same manner as mentioned above, except that the fermentation was stopped 5.5 days after the initiation of the fermentation. A part of the resulting fermentation product was immediately subjected to removal of alcohol at 20 °C under a reduced pressure of 15 mmHg, using a rotary evaporator, to an extent that the alcohol concentration of the cultured broth was decreased to 0.5 % by volume to obtain an antianaemic composition. Separately, 400 ml of the cultured broth was separated into a supernatant and a precipitate by centrifugation at 10,000 rpm for 10 min. The supernatant was concentrated at 30 °C or lower to about one fifteenth in terms of volume. The obtained concentrate was subjected to chromatographic fractionation by means of Amberlite XAD2 (available from Rohm and Hass Co., U.S.A.) and separated into a nonadsorptive iron(II)-containing substance (Fraction A) and an adsorptive iron(II)-containing substance (Fraction B), as shown in Fig. 4. Fractions A and B both are well adsorbed in the living body but Fraction A is excellent in the absorbability as compared with Fraction A. The amount of Fraction B obtained from 400 ml of the cultured broth was 9.6 mg.

Experiment 1

To demonstrate the antianaemic activity of the organoiron(II) compound-containing composition according to the present invention, animal tests were conducted using a composition obtained by the method as mentioned above.

1) Preparation of Test samples:

(a) Organoiron(II) compound-containing antianaemic composition according to the present invention:

$^{59}FeCl_3$ was added to a solution of ferric chloride in 0.1N hydrochloric acid in such an amount as would give a specific redioactivity of 6.25 µCi/50 µgFe, and the pH of the mixture was adjusted to pH 3 by adding an aqueous 0.1N sodium hydroxide solution.    The resultant solution was added, instead of 242 mg of $FeCl_3 \cdot 6H_2O$ as indicated in Table 1-(1), to a synthetic culture medium as shown in Table 1-(1) in an amount of 50 ppm in terms of amount of iron.  Except the above-mentioned change with respect to $FeCl_3$, in substantially the same manner as in Example 1, anaerobic culturing was effected to obtain a cultured broth having an alcohol content of 0.5 % by volume.  The cultured broth was concentrated at 30 °C under reduced pressure to obtain test samples respectively having iron contents of 10 and 40 µgFe/ml.

(b)  Ferrous sulfate solution (Comparative)

Into a $^{59}FeCl_3$ solution with a specific radioactivity of 12.5 µCi/µgFe was added ascorbic acid in an amount of 0.05 µmol  per µg of iron to give divalent iron.  By mixing this with an aqueous ferrous sulfate solution, comparative test samples respectively having 5 µCi/10 and 40 µgFe/ml, and having a pH value of 3 were obtained.

(c) Ferric chloride solution (Comparative)

Into the $^{59}FeCl_3$ solution was added an aqueous ferric chloride solution, and the pH of the mixture was adjusted to pH 3 by adding an aqueous sodium hydroxide solution. Comparative test samples respectively having 5 µCi/10 and 40 µgFe/ml were obtained.

(d) Mixture of a cultured broth from synthetic medium with no iron compound incorporated therein and ferrous sulfate (Comparative)

A cultured both prepared in the same manner as described in Example 1 except that $FeCl_3$ was not added was concentrated to 1/4 in volume. By mixing this with a ferrous sulfate solution (prepared according to the abovementioned procedures and having 10 µCi/80 µgFe/ml) at a ratio of 1:1 by volume, a comparative test sample having 5 µgCi/40 µgFe/ml was obtained.

(e) Ferrous orotate solution (Comparative)

From an aqueous ferrrous orotate solution containing labelled iron $^{59}Fe$, a comparative test sample having 5 mCi/40 µgFe/ml was prepared.

2) Administration:

Five-week old male Sprague-Dawly rats (available from Nippon Kurea K.K., Japan) were subjected to preparatory breeding for one week. They were fed with a feed "CA-1" (containing iron in an amount of 31.5 mg/100 g, and manufactured and sold by Nippon Kurea K.K., Japan), and city water was given. During the preparatory breeding, weight increase was checked. Those rats having a body weight of about 160 g at the age of six weeks were subjected to the experiments. Through the period of experiments, the rats were bred in a metabolic cage. No feeding was done for 18 hours before administering a test sample, but distilled water was continued to be given. Each test sample prepared according to the above-mentioned procedures was administered to a group of five rats, directly into their stomach by means of stomach probe, in an amount of 1 ml/160 g body weight. Six hours after administering the test sample, feeding of feed "CA-1" was resumed.

3) Determining Radioactivity of In-vivo Sample:

After administering each test sample, feces and urine were taken at intervals of hours to determine radioactivities thereof. 50 µl of blood was taken by means of a heparin-treated capillary from the tail vein at intervals of hours, and subjected to centrifugation at 3,000 rpm for 5 minutes to separate blood corpuscles from plasma. Radioactivities of the so obtained blood corpuscles and plasma were determined.

Absorption of iron into the body and incorporation of the body-absorbed iron into hemoglobin are shown in Table 4.

## Table 4

(Comparison between an organoiron(II)-containing composition of the present invention, an inorganic ferrous compound, an inorganic ferric compound and ferrous orotate, with respect to absorbability into the living body and incorporation of iron into hemoglobin)

| Iron concentration in sample ($\mu g/ml$) | Dose (Fe $\mu g/$ Kg·body weight) | Kind of sample | A Absorbability (%) | B Incorporation into Hemoglobin (%) |
|---|---|---|---|---|
| 10 | 62.5 | (a) | $70.2 \pm 2.1$ | $48.1 \pm 1.5$ |
|  |  | (b) | $67.4 \pm 3.6$ | $47.5 \pm 1.8$ |
|  |  | (c) | $50.1 \pm 4.2$ | $32.0 \pm 6.2$ |
| 40 | 250 | (a) | $55.1 \pm 8.1$ | $28.5 \pm 2.3$ |
|  |  | (b) | $37.6 \pm 4.0$ | $20.0 \pm 1.9$ |
|  |  | (c) | $29.9 \pm 6.9$ | $15.7 \pm 4.2$ |
|  |  | (d) | $31.0 \pm 3.5$ | $17.9 \pm 3.2$ |
|  |  | (e) | $36.5 \pm 3.5$ | $25.6 \pm 4.0$ |

Note

A (absorbability) = 100 x (1 - amount of discharged $^{59}Fe$/amount of total dose of $^{59}Fe$); and

B (Indorporation into Hemoglobin): $^{59}Fe$ content in the

- 32 -

0142790

whole      blood at the time of 168 hours after administration of a test sample.

Each figure represents a mean value ± standard error.

As is apparent from Table 4, when the iron concentration in an administered sample of the present composition is as low as about 1 mg/liter or less, there is not a remarkable difference in absorbability into a living body between the organic iron complex according to the present invention and the inorganic iron salts. This is assumed to be attributed to the fact that such an iron concentration as low as about 1 mg/liter or less is within such a range that the reducing capacity of the living body itself is sufficient therefor. But, when the iron concentration is relatively high, the antianaemic composition according to the present invention exhibits a remarkably improved absorbability into the living body as compared with $FeSO_4$, $FeCl_3$ and ferrous orotate. On the other hand, no significant difference in absorbability into the living body is seen between the mixutre of concentrated ordinary wine and ferrous sulfate [i.e. (d) in Table 4] and ferrous sulfate per se [i.e. (b) in Table 4].

Experiment 2

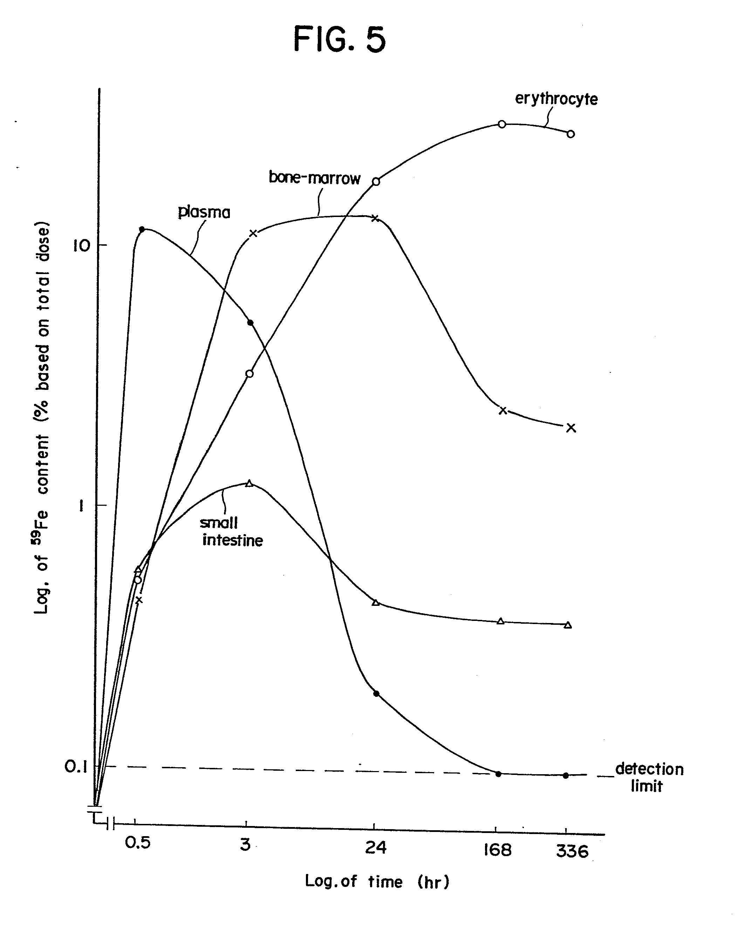

From the supernatant obtained in Example 1, a test sample having 250 μgFe/ml was prepared. In substantially the same manner as in Experiment 1, items 2) and 3), the

- 33 -

test sample was administered to rats in an amount of 250 µg Fe/Kg·body weight, and the changes in iron contents at the plasma, bone-marrow, erythrocyte and small intestine were determined with the lapse of time after the administration. The test results were shown in Fig.5, wherein the ordinate and abscissa respectively indicate the logarithm of each $^{59}$Fe content(%) based on the total dose and the logarithm of the time (hr) after administaration of the test sample.

As is clearly seen from Fig.5, first, the iron content in the plasma repidly increases but, in turn, rapidly decreases to reach nearly zero about 168 hours after the administration. Instead, second, the iron content in the bone-marrow increases and begins to decrease about 20 hours after the administration. Third, the iron content in the erythrocyte gradually increases to reach a plateau region (in which the iron content is larger than those observed with respect to the plasma and the bone-marrow) and the iron content in the erythrocyte which has once reached the plateau region does not change for about 50 days. The graphs suggest that the iron values absorbed in the plasma are transferred to the erythrocyte through the bone-marrow. The graphs also show that the organoiron(II) compound of the present antianaemic composition is very stably absorbed into the living body and that the incorporation of iron into hemoglobin is very large.

Therefore, the times of dosage can advantageously be reduced.

Comparative Example 1

substantially the same procedures as in Example 1 were repeated except that a cotton stopper was used instead of a fermentation stopper. The amount of iron in Fraction B obtained from 400 ml of the cultured broth was 4.6 mg.

Comparative Example 2

Substantially the same procedures as in Example 1 were repeated except that a cotton stopper was used instead of a fermentation stopper, and that potassium sodium tartrate and calcium chloride were not added to the synthetic culture medium. The amount of iron in Fraction B obtained from 400 ml of the cultured broth was 3.6 mg.

The curve(2) in Fig.1 shows the change in pH value of the culture medium relative to the fermentation period, the curve(2) in Fig.2 the growth of Saccharomyces cerevisiae OC-2 in the culture medium relative to the fermentation period, and the curve(2) in Fig.3 the change in organoiron(II) compound concentration, in terms of amount of iron, in the culture medium relative to the fermentation period.

Example 2

Substantially the same procedures as in Example 1 were repeated except that instead of the synthetic culture medium, a semi-synthetic culture medium of Table 2 to which 4.6 g of sodium citrate was added was employed. The amount of iron in Fraction B obtained from 400 ml of the cultured broth was 6.4 mg.

The Fraction B thus obtained, like that obtained in Example 1, was stable and had an excellent absorbability for a living body.

Example 3

Substantially the same procedures as in Example 1 were repeated except that Candida krusei K-503 was used instead of Saccharomyces cerevisiae OC-2. The amount of iron in Fraction B obtained from 400 ml of the cultured broth was 8.4 mg.

The Fraction B thus obtained, like those obtained in Example 1 and Example 2, was stable and had an excellent absorbability for a living body.

What is claimed is:

1. A process for producing an organoiron(II) compound-containing antianaemic composition which comprises subjecting a yeast to anaerobic culturing in a saccharide-containing nutrient medium therefor in the presence of an iron compound to form a cultured broth comprising an organoiron(II) compound, alcohol and water.

2. A process according to claim 1, wherein the nutrient medium contains an organic acid salt.

3. A process according to claim 2, wherein the nutrient medium further contains calcium chloride.

4. A process according to any one of claims 1 to 3, wherein the nutrient medium is a semi-synthetic culture medium or a synthetic culture medium.

5. A process according to any one of claims 2 to 4, wherein the organic acid salt is non-toxic.

6. A process according to claim 5, wherein the organic acid salt is at least one member selected from the group consisting of malic acid salts, citric acid salts, tartaric acid salts and succinic acid salts.

7. A process according to any one of claims 2 to 6, wherein the organic acid salt concentration of the nutrient medium is in the range of from about 0.1 to about 1.0 % by weight.

8. A process according to any one of claims 3 to 7, wherein the calcium chloride concentration of the nutrient medium is in the range of from about 0.001 to about 1.0 % by weight in terms of $CaCl_2 \cdot 2H_2O$.

9. A process according to any one of claims 1 to 8, wherein the anaerobic culturing is conducted in a culture vessel sealed with a fermentation stopper.

10. A process according to any one of claims 1 to 9, wherein the yeast is of a member selected from the group consisting of the genera Saccharomyces, Metschnikowia and Candida.

11. A process according to any one of claims 1 to 10, wherein the yeast is at least one member selected from the group consisting of Saccharomyces cerevisiae, Metschnikowia pulcherrima, Saccharomyces cheralieri, Saccharomyces bayanus, Saccharomyces italicus, Saccharomyces fermentati, Saccharomyces rosei, Saccharomyces heterogenicus and Candida krusei.

12. A process according to any one of claims 1 to 11, wherein the anaerobic culturing is conducted in the nutrient medium having a yeast population of $3 \times 10^5$ to $3 \times 10^6$/ml of the medium.

13. A process according to any one of claims 1 to 12, wherein the iron compound is at least one member selected from the group consisting of ferric chloride, ferrous sulfate, ferric sulfate, iron citrate and iron tartrate.

14. A process according to any one of claims 1 to 13, wherein the iron compound is incorporated into the nutrient medium in an amount of about 10 to about 70 ppm in terms of amount of iron.

15. A process according to any one of claims 1 to 14, wherein the saccharide content of the nutrient medium is about 5 to about 25 % by weight.

16. A process according to any one of claims 1 to 15, wherein the anaerobic culturing is conducted at a temperature of about 18 to about 30°C.

17. A process according to any one of claims 1 to 16, which further comprises removing the alcohol from the cultured broth to an extent that the resulting cultured broth has an alcohol content of less than about 1 % by volume.

18. A process according to any one of claims 1 to 16, which further comprises subjecting the cultured broth to separation to isolate a supernatant comprising an organoiron(II) compound, alcohol and water, and removing the alcohol from the supernatant to an extent that the resulting supernatant has an alcohol content of less than about 1 % by volume.

19.  A process according to any one of claims 1 to 16, which further comprises concentrating the cultured broth to from about 1/10 its volume up to dryness.

20.  A process according to any one of claims 1 to 16, which further comprises subjecting the cultured broth to separation to isolate a supernatant comprising an organoiron (II) compound, alcohol and water, and concentrating the isolated supernatant to from about 1/10 its volume up to dryness.

21.  An organoiron(II) compound-containing antianaemic composition produced by a process comprising subjecting a yeast to anaerobic culturing in a saccharide-containing nutrient medium therefor in the presence of an iron compound to form a cultured broth comprising an organoiron(II) compound, alcohol and water.

22.  An organoiron(II) compound-containing antianaemic composition produced by a process according to any one of claims 2 to 20.

$\frac{1}{5}$

# FIG. I

FERMENTATION PERIOD, days

FIG. 2

# FIG. 3

FERMENTATION PERIOD, days

# FIG. 4

# FIG. 5